# EUROPEAN PATENT SPECIFICATION

(11) **EP 3 439 719 B1**
(45) Date of publication and mention of the grant of the patent: **03.06.2020**
(21) Application number: 17715163.6
(22) Date of filing: 04.04.2017
(51) Int. Cl.: A61M 15/00

(54) **A METHOD OF COUNTING MEDICAMENT DOSES DISPENSED BY AN INHALER - AND SYSTEM FOR THE SAME**
VERFAHREN ZUM ZÄHLEN VON ÜBER EINEN INHALATOR ABGEGEBENEN ARZNEIMITTELDOSEN - UND SYSTEM DAFÜR
PROCÉDÉ DE COMPTAGE DES DOSES DE MÉDICAMENT DISTRIBUÉ PAR UN INHALATEUR - ET SYSTÈME À CET EFFET

(30) Priority: 05.04.2016 EP 16163914
(43) Date of publication of application: 13.02.2019
(73) Proprietor: Chiesi Farmaceutici S.p.A., 43122 Parma (IT)
(72) Inventor: HARRIS, David Stuart, 43122 Parma (IT); MITCHELL, Colin Roy, 43122 Parma (IT); CANNER, Philip David, 43122 Parma (IT)
(74) Representative: PGA S.p.A.
(86) International application number: PCT/EP2017/058000
(87) International publication number: WO 2017/174588

(56) References cited:
- WO-A1-2011/089490
- US-A1- 2006 065 713
- US-A1- 2010 252 036
- US-A1- 2012 174 914
- US-A1- 2012 304 990
- US-A1- 2016 082 208

## Description

### FIELD OF THE INVENTION

The present invention relates to a method of counting medicament doses dispensed by an inhaler; an electronic device for counting medicament doses dispensed by the inhaler; a system comprising the inhaler and the electronic device and a container, such as a pressurised metered dose inhaler canister or a dry powder reservoir, comprising the medicament to be administered by the inhaler.

### BACKGROUND OF THE INVENTION

Inhalers are well known for providing medicament to patients. Well known inhaler types include pressurised metered dose inhalers (pMDls) and dry powder inhalers (DPIs). They each have a container including a medicament. A user uses a pMDI to deliver an amount of an aerosolized medicament from the container to their lungs. A user uses a DPI to deliver medicament from the container or reservoir in the form of a dry powder to their lungs. Both types of inhaler are used for treating asthma, chronic obstructive pulmonary disease (COPD) as well as other conditions.

Effective treatment of many conditions using inhalers of these types requires the regular administration of a dose of medicament. An inhaler, and in particular the medicament container of an inhaler, provides only a limited number of doses. It is useful to count the number of doses so that a user knows when an inhaler will run out of medicament to deliver to a user. In regulated markets, a dose counter is mandatory for bronchodilators, and other rescue medication that patients take in the event of an exacerbation.

Many mechanical arrangements are known for counting medicament doses provided by an inhaler. One example is described in international patent application with publication No. WO 98/28033 in the name of Norton Healthcare Limited and it is illustrated in Figures 1 to 3 in which like features have been given like reference numerals. The inhaler dose counting mechanism 10 illustrated uses a pawl and ratchet mechanism 12 that is stepwise rotated each time canister 14 with medicament is pushed down to rotate a counter 16. As with this type of mechanical arrangement generally, this example is complex and bulky. This type of mechanism adds significantly to the cost of an inhaler.

Many "smart" inhalers are known that provide electronic communication from an inhaler to a computer such as a smartphone, tablet, laptop or desktop computer.

These arrangements include the Smartinhaler Platform from Adherium that is used with inhalers provided by GSK and Boehringer Ingelheim; the SmartTurbo2 and Turbu+ systems again provided by Adherium that integrates with inhalers from AstraZeneca; Propeller Health's Propeller Sensor; Gecko Health's arrangement called CareTRx; Cohero Health's HeroTracker and Spirothor products; LifeMap Solutions' product that communicates with their Asthma Health application; and Amiko's Amiko Smart Tracker device. These many arrangements all use Bluetooth (registered trade mark) wireless technology for communication between inhaler and computer. Bluetooth is a standard for exchanging data between electronic devices over short distances using short-wavelength radio waves. Both the transmitter and receiver of a pair of devices communicating using Bluetooth require an electrical power source either from a battery or mains supply. "Classic" Bluetooth has quite high power requirements and the more recent version called Bluetooth Low Energy has lower power requirements, but still requires an appreciable power supply. Thus, all of these types of "smart" inhaler require a battery as a power supply. This impacts considerably on the cost of providing a "smart" inhaler even though in these arrangements the "smart" Bluetooth transceiver part of the inhaler is provided in a detachable and reusable portion. Document WO 2011/089490 discloses an aerosol delivery system with a sensor operatively connected to a controller via wireless communication.

### BRIEF SUMMARY OF THE INVENTION

The inventors of the arrangement described herein have appreciated that a "smart" inhaler that communicates with a computer such as a smartphone, tablet or smartwatch or other separate electronic device can be provided using a near field communication (NFC) tag attached to an inhaler for the communication. In the embodiments described herein, the inhaler is used for counting medicament doses provided by the inhaler for which the use of NFC is particularly advantageous. Near field communication uses electromagnetic induction between an antenna of the NFC tag and an antenna of the electronic device communicating with it for communication between the NFC tag and the electronic device. The NFC tag does not have or require its own power supply. Power is provided from the electronic device (for example, the smartphone or smartwatch) communicating with the tag. In this way, a simple and low cost "smart" inhaler is provided particularly for medicament dose counting. Compared to a Bluetooth arrangement, this arrangement is more secure, smaller and no time-consuming pairing is required (where Bluetooth devices form a communication connection with one another) between the inhaler and electronic device. Additionally, it is significantly lower cost and physically smaller in size. It could even be utilised within a stick-on label, for example.

In the arrangements described below, the inhaler comprising a NFC tag is located near to or touched against a separate electronic device to which an appropriate software application is installed. Each time a user takes a medicament dose from the inhaler, the inhaler is located near to or touched against the separate electronic device and a dose count is incremented by one. The dose counter may be provided by a store or memory of the NFC tag or by a store or memory of the electronic device. While the inventors of the arrangement have appreciated that this arrangement may not give a completely accurate measure of the dose count if the user fails to locate the inhaler near to or touch it against the separate electronic device after every dose, the inventors have appreciated that such a dose counter is good enough for many purposes and provides surprisingly useful data despite the simplicity of the device.

The invention in its various aspects is defined in the independent claims below to which reference should now be made. Advantageous features are set forth in the dependent claims.

According to the present invention it is provided a method of counting medicament doses dispensed by an inhaler, the inhaler comprising a passive electronic tag for communication with a separate electronic device, the method comprising: locating the inhaler near or touching the inhaler against the separate electronic device such that communication is established between the passive electronic tag and the separate electronic device; and in response, the separate electronic device incrementing a dose counter associated with the inhaler.

In a preferred embodiment the passive electronic tag comprises a near field communication tag, possibly comprising a store. Such store can include one or more of the following: an identifier of the inhaler, the dose counter, a location associated with the inhaler, wherein the location can comprise position of registration of the inhaler; also the store can include storing time of registration of the inhaler.

The separate electronic device can comprise a computer, and is selected from a smartphone, a laptop computer, a desktop computer, a tablet computer, a smartwatch or another electronic wearable device. In an embodiment the separate electronic device includes the dose counter.

In an embodiment the inhaler is a pressurised metered dose inhaler (pMDI) or a dry powder inhaler (DPI).

In an embodiment, in response to the fact that the inhaler is located near to or touched against the separate electronic device, the said separate electronic device also provides data input means for inputting data into the separate electronic device associated with the inhaler.

The inhaler can comprise a container, selected from a pMDI canister or a reservoir of a DPI, both including the medicament and wherein the passive electronic tag is located on the container.

According to another aspect of the present invention it is provided an electronic device for counting medicament doses dispensed by an inhaler, the electronic device comprising: a passive electronic tag reader; and the electronic device being configured such that, in use: when an inhaler comprising a passive electronic tag that can form a communication connection with the electronic device is located near to, or touching the electronic device, communication is established between the passive electronic tag and the passive electronic tag reader; and the electronic device increments a dose counter associated with the inhaler. The electronic device can further comprise one or more components for implementing the method steps described above.

Also, the present invention provides a system comprising: an inhaler including a passive electronic tag for communication with an electronic device; and an electronic device including: a passive electronic tag reader; and the electronic device being configured such that, in use: when an inhaler comprising a passive electronic tag that can form a communication connection with the electronic device is located near to or touching the electronic device, communication is established between the passive electronic tag and the passive electronic tag reader; and the electronic device increments a dose counter associated with the inhaler. The system can further comprise one or more components for implementing the method steps described above.

Also provided is a container comprising a medicament for an inhaler, the container comprising a passive electronic tag for communication with an electronic device; the container being configured such that, in use: when the container is located near to or touching the electronic device, communication is established between the passive electronic tag and the passive electronic tag reader; and the electronic device increments a dose counter associated with the container. The container can be adapted to be used in the system defined above. An inhaler, including the container is also provided.

According to a further aspect of the present invention a computer program comprises computer program code means which, when run on a computer, implement the method steps described above.

Arrangements are described in more detail below and take the form of a method of counting medicament doses dispensed by an inhaler, the inhaler comprising a passive electronic tag for communication with a separate electronic device. The method comprises: locating the inhaler near or touching the inhaler against the separate electronic device such that communication is established between the passive electronic tag and the separate electronic device. In response, the separate electronic device increments the dose counter associated with the inhaler.

### BRIEF DESCRIPTION OF THE DRAWINGS

The invention will be described in more detail, by way of example, with reference to the accompanying drawings, in which:

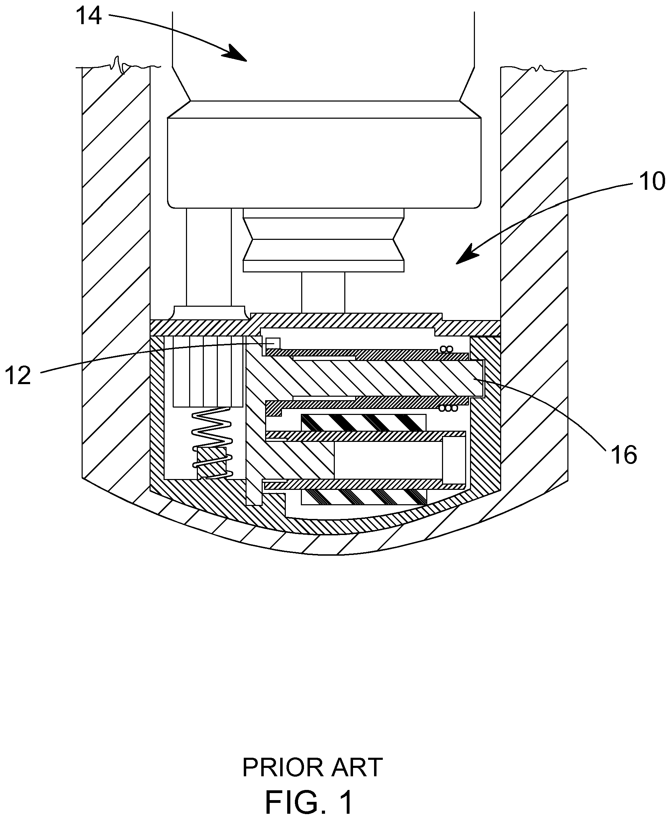

Figure 1 (prior art) is a cross-sectional view through a pMDI showing a counter mechanism;

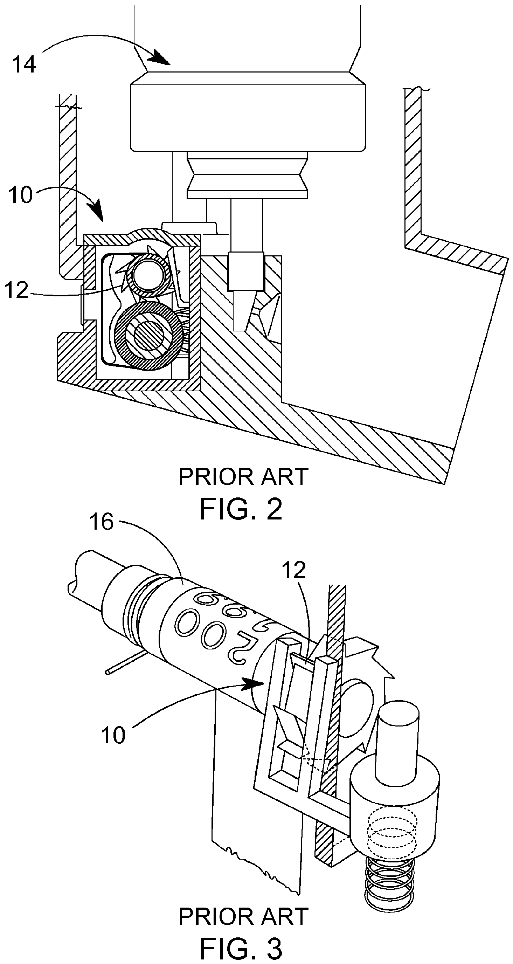

Figure 2 (prior art) is a cross-sectional view taken at right angles to the view of Figure 1;
Figure 3 (prior art) is a schematic perspective view of part of the pMDI of Figures 1 and 2;
Figure 4 is a perspective view of a pMDI for use in the method embodying an aspect of the present invention;
Figure 5 is a cross section through the inhaler of Figure 4 for use in the method embodying an aspect of the present invention;
Figure 6 is a schematic diagram illustrating the method embodying an aspect of the present invention; and
Figure 7 is a cross section through a pMDI embodying an aspect of the present invention.

### DETAILED DESCRIPTION OF THE INVENTION

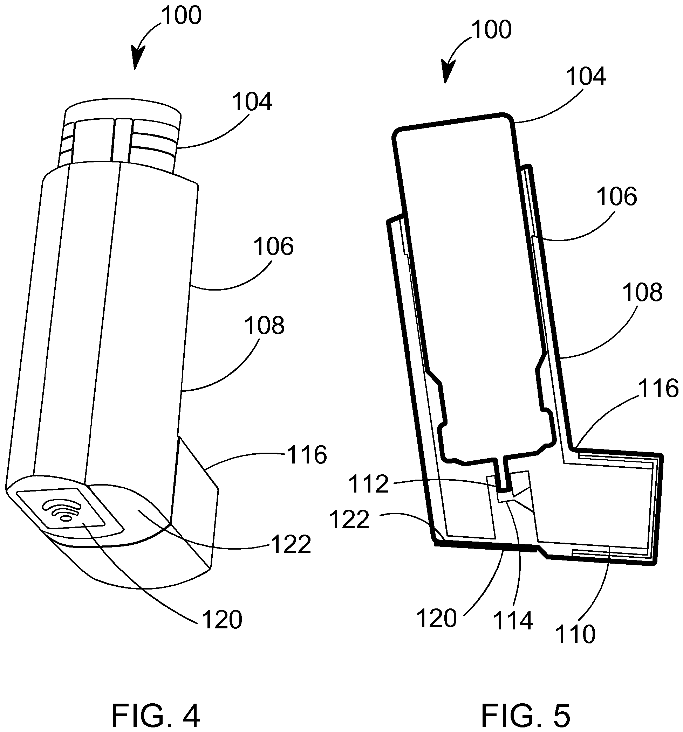

An example method of counting medicament doses dispensed by an inhaler 100 will now be described with reference to Figures 4 to 6 as well as an electronic device 102 for counting medicament doses dispensed by the inhaler and a container, such as a canister, for the inhaler. Like features in Figures 4 to 6 have been given like reference numerals. The features of Figures 4 to 7 relate to an inhaler provided with a NFC tag which is a pMDI. The same NFC, with same functionality, can also be applied to an inhaler represented by a multidose DPI, which delivers multiple, single doses of a medicament powder from a reservoir or, in alternative, also by a single-dose DPI that delivers single doses of a medicament powder packaged separately. These kinds of inhalers are all included in the present invention.

The NFC tag may be attached on the external surface of the inhaler (pMDI or DPI) or on the external surface of the medicament container which is the aerosol canister for a pMDI or the medicament reservoir for a multidose DPI.

The inhaler 100 of Figures 4 and 5 is a pMDI largely of known arrangement. It includes a container in the form of a canister 104 containing medicament to be released in aerosol form. The canister is housed in a housing portion 106 of a plastics casing 108. The casing also includes a mouthpiece 110 for inserting into a user's mouth that extends laterally to the housing portion. An outlet 112 of the canister, through which medicament is released from the canister, is located within a fluid dispenser 114. The fluid dispenser is arranged such that as the outlet of the canister is pushed against it, fluid is released from the canister laterally to the canister and out through the mouthpiece. In Figures 4 and 5, a cap 116 is shown covering the mouthpiece.

To dispense medicament from the inhaler, the cap 116 is removed by a user. The mouthpiece 110 is then inserted by a user into his mouth. The user then presses the canister 104 against the fluid dispenser 114 and a dose of medicament is released from the outlet 112 of the canister and the dispensed medicament passes laterally from the outlet of the canister through the mouthpiece, into the user's mouth and onwards to their lungs.

Significantly, the inhaler 100 of Figures 4 and 5 also includes an NFC tag or label 120 . The NFC tag is located on the outside of the inhaler on the casing 108 and, in particular, on a base 122 of the housing portion 106 of the casing. The NFC tag is located adjacent the mouthpiece 110 and facing the canister 104. The NFC tag is attached to the casing by adhesive. The NFC tag is a passive electronic tag. It is called a passive electronic tag because it does not have its own power supply. The NFC tag has an antenna (not shown). It uses electromagnetic induction between this antenna and an antenna of an NFC tag reader of the electronic device communicating with it for communication between the NFC tag and the NFC tag reader of the electronic device. The NFC tag has stored in it a unique identifier to uniquely identify the inhaler. While an NFC tag is illustrated, a different passive electronic tag in the form of another radio frequency identification (RFID) tag could be provided.

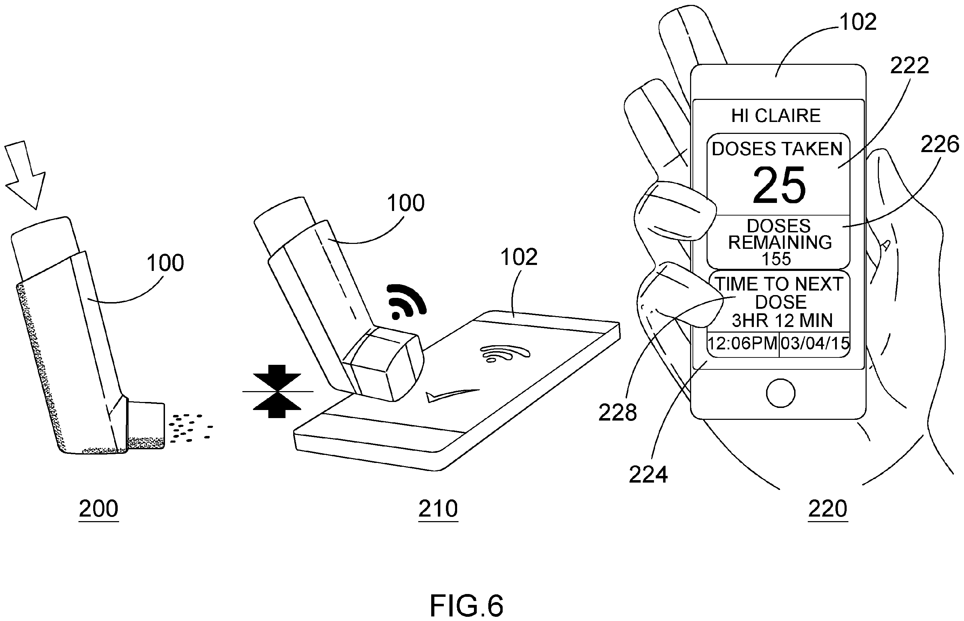

Figure 6 illustrates the method of counting medicament doses dispensed by the inhaler 100 using a separate electronic device 102 comprising a passive electronic tag reader in the form of an NFC tag reader, and dose counter implemented in software. In this example, the separate electronic device is a smartphone. However, the electronic device may comprise another type of computer including a passive electronic tag reader and appropriate software, such as a laptop computer, desktop computer, tablet computer, smartwatch or other electronic wearable device including a display.

Firstly, as illustrated at step 200, a user takes a dose of medicament from the inhaler 100 as described above. As illustrated at the next step, step 210, the user then locates the inhaler near the smartphone 102 or touches the inhaler against the smartphone. Communication is then established between the NFC tag 120 and the NFC tag reader of the smartphone. In response, as illustrated at step 220, the smartphone increments the dose counter by one as illustrated by the indication of the number of doses taken 222 displayed on the display 224 of the smartphone.. The smartphone display also displays other useful data such as the doses remaining 226 and the time until the next dose needs to be taken 228.

The doses remaining is initiated by the user when the user has a new inhaler 100 based on the expected number of doses from the inhaler. As mentioned above, each NFC tag 120 has stored in it a unique identifier for the inhaler. When a user locates the inhaler near the smartphone 102 or touches the inhaler against the smartphone for the first time, the smartphone asks the user if this is a new inhaler that they are to use. If the user responds that it is new, the smartphone uses the unique identifier provided by the NFC tag to identify the inhaler type and its known total number of doses based on data stored in the smartphone or in a remote database (not illustrated) in communication with the smartphone. The total number of doses is then stored in the smartphone's memory. The smartphone then decrements this number by one every time that the dose counter is incremented by one and the new number of doses remaining is stored in the smartphone's memory and displayed on the smartphone's display 224. Rather than the identity of the inhaler being used to obtain information on the total number of doses that can be provided by the inhaler, the NFC tag may store the number of doses that can be delivered and this information can be provided to the smartphone directly.

The NFC tag 120 is also used to provide the time between doses. In a similar way to how the total number of doses is provided described above, the time between doses may be provided to the smartphone 102 based on the identity of the inhaler as provided by the NFC tag and then by interrogation of a database that holds the time between doses for the identified inhaler where the database is either stored on the smartphone or remotely. The NFC tag may also store the time between doses on it and this may be provided directly to the smartphone the first time that the inhaler communicates with the smartphone.

Significantly, the use of a passive electronic tag means that the smartphone does not need to connect to a remote computer server based or cloud based service in order to obtain relevant information.

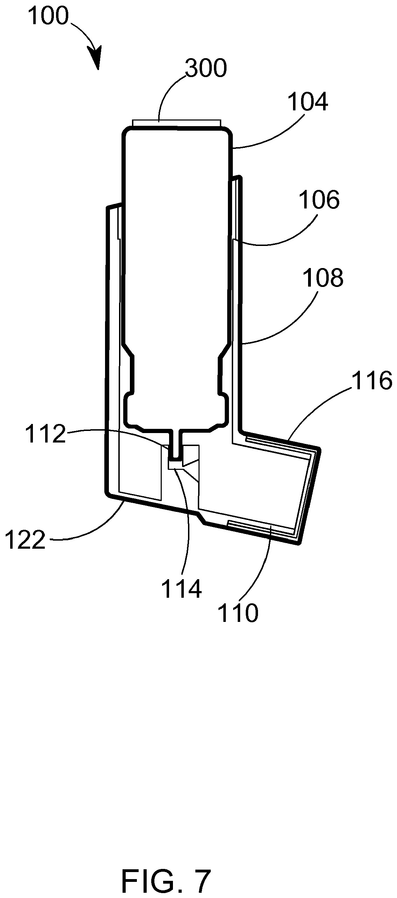

An alternative arrangement to that of Figures 4 to 6 is illustrated in Figure 7. The arrangement of Figure 7 is similar in most respects to that illustrated in Figures 4 to 6, the significant difference between arrangements can be seen by comparing Figure 5 to Figure 7.

In the arrangement of Figure 7, the passive electronic tag or NFC tag 300 is located on the container (or canister) 104 of the inhaler (pMDI) 100 rather than on the casing 108 or housing of the inhaler as in the arrangement of Figure 5. In particular, the NFC tag is located on the exposed outer portion of the canister that is pressed to dispense a medicament dose. This is significant because it means that the NFC tag is associated with a canister of the inhaler (that is not reusable once empty) rather the inhaler casing or housing (which is reusable). In this way, a new NFC tag is provided whenever a new canister is used.

Another difference of the arrangement of Figure 7 over that of Figures 4 to 6, is that the store or memory of the NFC tag 300 includes the dose counter rather than the separate electronic device (although the arrangement of Figures 4 to 6 could also have the dose counter located on the NFC tag rather than in the smart phone). The NFC tag obtains power through electromagnetic induction between an antenna of the NFC tag and an antenna of the electronic device communicating with it and this is sufficient for the store or memory of the NFC tag to act as a dose counter. Thus, even in this arrangement, the NFC tag does not have or require its own power supply. The counter is incremented, as described above, with reference to Figures 4 to 6 by locating the NFC tag of the inhaler near the electronic device or touching the inhaler against the electronic device.

In this example, the store can store 16 bits or up to a count of 65,535 doses. The store is initialised to 0 by the manufacturer (that is, all of the 16 bits are set to 0) and this indicates a new canister or inhaler which has not provided a medicament dose.

The store of the NFC tag may store a location associated with the inhaler or canister. This may be, for example, a representation of the user's address such as their house number and postcode so that by a third party interrogating the NFC tag this information may be ascertained. In this way, if a lost inhaler or canister is found it can be returned to the user's address. Another identifier of the user could be used, such as a telephone number or e-mail address.

The location may also or alternatively include a position of registration of the inhaler or canister. That is to say, the position or location at which the inhaler or canister is first in communication connection with a separate electronic device, such as a smartphone. The location may be provided directly from the smartphone such as from its Global Positioning System (GPS) sensor. In this arrangement, it is assumed that the position or location of first use of the inhaler or canister is their home or other location where the user could be readily contacted. This is a very user-friendly feature.

The store (either in the NFC tag or smart phone) may also store a time of registration of the inhaler or canister. This time can be ascertained by the smartphone when in close proximity to the NFC tag. The smartphone can have stored on it the shelf life or usable lifetime of the inhaler or canister or alternatively this could be stored in the store of the NFC tag. Thus, by comparing the time of registration or first use of the inhaler or canister with the shelf life, the smart phone can provide an indication or warning to the user that the inhaler or canister has expired (or is close to expiry) and should not be used (or should be replaced soon).

The store (of the NFC tag or separate electronic device) may also store time and location pairs of when and where the inhaler is used. The NFC tag has capacity to store, for example, two or three time location pairs.

A further alternative arrangement includes a microprocessor, located on or into the inhaler inhaler (pMDI or DPI) which may store, update and/or write on the NFC tag additional information related, among others, to localisation, time, inhalation flow of the patient during the administration for communication with a separate electronic by locating near or touching the inhaler against the separate electronic device such that communication is established.

The separate electronic device or smartphone may provide a data input means for inputting data into it associated with the inhaler in response to the NFC tag of the inhaler or canister being located near the electronic device or touching the inhaler against the electronic device. This allows a user to enter notes regarding their use of the inhaler or how they are feeling. This can be reviewed, for example, to see the effect if a user has previously missed a dose or doses. A condition management application, which is more sophisticated, may alternatively be launched by the electronic device in response to the NFC tag of the inhaler or canister being located near the electronic device or touching the inhaler against the electronic device. Such an application may ask the user particular questions about how they are feeling or use of the inhaler again so the effect of the inhaler can be ascertained.

Information entered by the user may be uploaded from the electronic device to a remote computer server or servers, or cloud. This information may be used by the inhaler or canister manufacturer or research institution to monitor the efficacy of the inhaler or canister or the user's use of it.

The illustrations described above have focused on the MDI as the electronically enabled inhaler. The features are equally applicable to a multidose DPI that delivers multiple single doses of a medicament from a reservoir or, in alternative, also to a single-dose DPI that delivers single doses of a medicament packaged separately.

Embodiments of the present invention have been described. It will be appreciated that variations and modifications may be made to the described embodiments within the scope of the present invention.

## Claims

1. A method of counting medicament doses dispensed by an inhaler, the inhaler comprising a passive electronic tag for communication with a separate electronic device, the method comprising:
locating the inhaler near or touching the inhaler against the separate electronic device such that communication is established between the passive electronic tag and the separate electronic device; and
in response, the separate electronic device incrementing a dose counter associated with the inhaler.

2. A method according to claim 1, wherein the passive electronic tag comprises a near field communication tag.

3. A method according to claim 1 or 2, wherein the separate electronic device comprises a computer, and is selected from a smartphone, a laptop computer, a desktop computer, a tablet computer, a smartwatch and an electronic wearable device.

4. A method according to any preceding claim, wherein the inhaler is a pressurised metered dose inhaler or a dry powder inhaler.

5. A method according to any preceding claim, wherein the passive electronic tag comprises a store.

6. A method according to claim 5, wherein the store includes an identifier of the inhaler.

7. A method according to claim 5 or claim 6, wherein the store includes the dose counter.

8. A method according to any preceding claim, wherein the separate electronic device includes the dose counter.

9. A method according to any of claims 6 to 8, wherein the store stores a location associated with the inhaler.

10. A method according to claim 9, wherein the location comprises position of registration of the inhaler.

11. A method according to any of claims 5 to 10, wherein the store stores time of registration of the inhaler.

12. A method according to any preceding claim, wherein in response to the fact that the inhaler is located near to or touched against the separate electronic device, the said separate electronic device also provides data input means for inputting data into the separate electronic device associated with the inhaler.

13. A method according to any preceding claim, wherein the inhaler comprises a container, selected from a pMDI canister or a reservoir of a DPI, both including the medicament and wherein the passive electronic tag is located on the container.

14. A method according to any preceding claim, wherein the passive electronic tag is attached to an external surface of the inhaler.

15. A system comprising:
an inhaler including a passive electronic tag for communication with an electronic device; and,
an electronic device configured for counting medicament doses dispensed by an inhaler, the electronic device comprising:
a passive electronic tag reader; and
the electronic device being configured such that, in use:
when the inhaler comprising a passive electronic tag that can form a communication connection with the electronic device is located near to or touching the electronic device, communication is established between the passive electronic tag and the passive electronic tag reader; and the electronic device increments a dose counter associated with the inhaler.

## Patentansprüche

1. Verfahren zum Zählen von über einen Inhalator abgegebenen Arzneimitteldosen, wobei der Inhalator ein passives elektronisches Identifizierungskennzeichen zur Kommunikation mit einer separaten elektronischen Vorrichtung aufweist, wobei das Verfahren aufweist:
Anordnen des Inhalators in der Nähe der separaten elektronischen Vorrichtung oder Anlegen des Inhalators an diese, so dass zwischen dem passiven elektronischen Identifizierungskennzeichen und der separaten elektronischen Vorrichtung Kommunikation hergestellt wird; und
in Antwort darauf, die separate elektronische Vorrichtung einen dem Inhalator zugeordneten Dosiszähler inkrementiert.

2. Verfahren nach Anspruch 1, wobei das passive elektronische Identifzierungskennzeichen ein Nahbereich-Kommunikations-Identifizierungskennzeichen aufweist.

3. Verfahren nach Anspruch 1 oder 2, wobei die separate elektronische Vorrichtung einen Computer aufweist und ausgewählt ist aus einem Smartphone, einem Laptop Computer, einem Desktop Computer, einem Tablet Computer, einer Smartwatch und einer elektronisch tragbaren Vorrichtung.

4. Verfahren nach einem vorhergehenden Anspruch, wobei der Inhalator ein unter Druckmessdosis-Inhalator oder ein Trockenpulverinhalator ist.

5. Verfahren nach einem vorhergehenden Anspruch, wobei das passive elektronische Identifikationskennzeichen einen Speicher aufweist.

6. Verfahren nach Anspruch 5, wobei der Speicher eine Kennung des Inhalators enthält.

7. Verfahren nach Anspruch 5 oder Anspruch 6, wobei der Speicher den Dosiszähler enthält.

8. Verfahren nach einem vorgehenden Anspruch, wobei die separate elektronische Vorrichtung den Dosiszähler enthält.

9. Verfahren nach einem der Ansprüche 6 bis 8, wobei der Speicher einen dem Inhalator zugeordneten Ort speichert.

10. Verfahren nach Anspruch 9, wobei der Ort eine Registrationsposition des Inhalators aufweist.

11. Verfahren nach einem der Ansprüche 5 bis 10, wobei der Speicher eine Registrationszeit des Inhalators speichert.

12. Verfahren nach einem vorhergehenden Anspruch, wobei in Antwort auf die Tatsache, dass sich der Inhalator in der Nähe der separaten elektronischen Vorrichtung befindet oder an diese angelegt ist, die separate elektronische Vorrichtung auch Dateneingabemittel bereitstellt, um Daten in die separate elektronische Vorrichtung in Zuordnung zu dem Inhalator einzugeben.

13. Verfahren nach einem vorhergehenden Anspruch, wobei der Inhalator einen Behälter aufweist, der ausgewählt ist aus einem pMDI-Kanister oder einem Reservoir eines DPI, die beide das Arzneimittel enthalten, und wobei das passive elektronische Identifizierungskennzeichen an dem Behälter angeordnet ist.

14. Verfahren nach einem vorhergehenden Anspruch, wobei das passive elektronische Identifizierungskennzeichen an einer Außenoberfläche des Inhalators angebracht ist.

15. System, welches aufweist:
einen Inhalator, der ein passives elektronisches Identifizierungskennzeichen zur Kommunikation mit einer elektronischen Vorrichtung aufweist; und
eine elektronische Vorrichtung, die konfiguriert ist, um von einem Inhalator abgegebene Arzneimitteldosen zu zählen, wobei die elektronische Vorrichtung aufweist:
ein Passives-Elektronisches-Identifizierungskennzeichen-Lesegerät; und
die elektronische Vorrichtung, die derart konfiguriert ist, dass im Gebrauch:
wenn der Inhalator, der ein passives elektronisches Identifizierungskennzeichen aufweist, das eine Kommunikationsverbindung mit der elektronischen Vorrichtung bilden kann, in der Nähe der elektronischen Vorrichtung angeordnet ist oder diese berührt, zwischen dem passiven elektronischen Identifizierungskennzeichen und dem Passives-Elektronisches-Identifizierungskennzeichen-Lesegerät Kommunikation hergestellt wird; und
die elektronische Vorrichtung einen dem Inhalator zugeordneten Dosiszähler inkrementiert.

## Revendications

1. Procédé de comptage de doses de médicament distribuées par un inhalateur, l'inhalateur comprenant une étiquette électronique passive pour la communication avec un dispositif électronique séparé, le procédé comprenant :
la localisation de l'inhalateur près de, ou le fait de toucher l'inhalateur avec le dispositif électronique séparé de sorte que la communication est établie entre l'étiquette électronique passive et le dispositif électronique séparé ; et
en réponse, le dispositif électronique séparé incrémentant un compteur de dose associé à l'inhalateur.

2. Procédé selon la revendication 1, l'étiquette électronique passive comprenant une étiquette de communication en champ proche.

3. Procédé selon la revendication 1 ou 2, le dispositif électronique séparé comprenant un ordinateur, et étant sélectionné parmi un smartphone, un ordinateur portable, un ordinateur de bureau, une tablette, une montre intelligente et un dispositif électronique portable.

4. Procédé selon l'une quelconque des revendications précédentes, l'inhalateur étant un inhalateur sous pression à dose mesurée ou un inhalateur de poudre sèche.

5. Procédé selon l'une quelconque des revendications précédentes, l'étiquette électronique passive comprenant un dispositif de stockage.

6. Procédé selon la revendication 5, le dispositif de stockage comprenant un identificateur de l'inhalateur.

7. Procédé selon la revendication 5 ou la revendication 6, le dispositif de stockage comprenant le compteur de dose.

8. Procédé selon l'une quelconque des revendications précédentes, le dispositif électronique séparé comprenant le compteur de dose.

9. Procédé selon l'une quelconque des revendications 6 à 8, le dispositif de stockage stockant une localisation associée à l'inhalateur.

10. Procédé selon la revendication 9, la localisation comprenant la position de l'enregistrement de l'inhalateur.

11. Procédé selon l'une quelconque des revendications 5 à 10, le dispositif de stockage stockant le temps d'enregistrement de l'inhalateur.

12. Procédé selon l'une quelconque des revendications précédentes, dans lequel en réponse au fait que l'inhalateur est localisé près de, ou touché par le dispositif électronique séparé, ledit dispositif électronique séparé fournit également un moyen de saisie de données pour entrer des données dans le dispositif électronique séparé associé à l'inhalateur.

13. Procédé selon l'une quelconque des revendications précédentes, l'inhalateur comprenant un récipient, sélectionné parmi une cartouche pMDI ou un réservoir d'un DPI, incluant tous deux le médicament et l'étiquette électronique passive étant localisée sur le récipient.

14. Procédé selon l'une quelconque des revendications précédentes, l'étiquette électronique passive étant fixée à une surface externe de l'inhalateur.

15. Système comprenant :
un inhalateur comprenant une étiquette électronique passive pour la communication avec un dispositif électronique ; et,
un dispositif électronique configuré pour compter les doses de médicament distribuées par un inhalateur, le dispositif électronique comprenant :
un lecteur d'étiquette électronique passive ; et
le dispositif électronique étant configuré de sorte que, durant l'utilisation :
lorsque l'inhalateur comprenant une étiquette électronique passive qui peut former une connexion de communication avec le dispositif électronique est localisé près de, ou touche le dispositif électronique, la communication est établie entre l'étiquette électronique passive et le lecteur d'étiquette électronique passive ; et le dispositif électronique incrémente un compteur de dose associé à l'inhalateur.
